(19)

Europäisches Patentamt
European Patent Office
Office européen des brevets

(11) **EP 1 783 213 A1**

(12) **EUROPEAN PATENT APPLICATION**

(43) Date of publication:
   **09.05.2007 Bulletin 2007/19**

(51) Int Cl.:
   *C12N 9/04* (2006.01)   *C07K 14/385* (2006.01)
   *C12Q 1/00* (2006.01)

(21) Application number: **06006698.2**

(22) Date of filing: **30.03.2006**

(84) Designated Contracting States:
   **AT BE BG CH CY CZ DE DK EE ES FI FR GB GR HU IE IS IT LI LT LU LV MC NL PL PT RO SE SI SK TR**
   Designated Extension States:
   **AL BA HR MK YU**

(30) Priority: **07.11.2005 JP 2005322046**

(71) Applicant: **Toyo Boseki Kabushiki Kaisha
   Osaka-shi
   Osaka-fu (JP)**

(72) Inventor: **Aiba, Hiroshi, co/ Tsuruga Inst. of Biotechn.
   Tsuruga-shi
   Fukui-ken (JP)**

(74) Representative: **Weber, Thomas et al
   Patentanwälte
   von Kreisler-Selting-Werner,
   Postfach 10 22 41
   50462 Köln (DE)**

Remarks:
Amended claims in accordance with Rule 86 (2) EPC.Pls Publish R.86(2) clms dated 18-01-2007 in addition to original clms.

(54) **Novel glucose dehydrogenase**

(57)   The invention relates to a eukaryote-derived glucose dehydrogenase that retains, after being heat-treated in a liquid state at 55°C for 15 minutes, at least 90% of its activity before the heat treatment; a process for producing the glucose dehydrogenase, comprising the steps of culturing a eukaryotic microorganism and extracting and purifying the glucose dehydrogenase; a method for measuring a glucose concentration using the glucose dehydrogenase; and an assay kit and glucose sensor comprising the glucose dehydrogenase.

**Description**

Field of the Invention

The present invention relates to novel glucose dehydrogenases (hereinafter glucose dehydrogenase is also referred to as GDH) that can be used for glucose measuring reagents and glucose sensors.

Background of the Invention

Self measurement of blood glucose is important for diabetic patients to know their usual blood glucose levels and use such levels efficiently for their treatment. Sensors for blood glucose self measurement employ enzymes that use glucose as a substrate. Examples of such enzymes include glucose oxidase (EC 1.1.3.4). Since glucose oxidase has the advantages of high specificity to glucose and excellent heat stability, it has been used for many years as an enzyme for blood glucose sensors, and the first report on glucose oxidase was published as many as 40 years ago. In blood glucose sensors employing glucose oxidase, measurement is made by transferring, to an electrode via a mediator, electrons generated in the process of oxidization of glucose into D-glucono-δ-lactone. Such sensors, however, have the problem of dissolved oxygen influencing the measurements, since glucose oxidase is likely to transfer protons generated by the reaction to oxygen.

To avoid such a problem, NAD(P)-dependent glucose dehydrogenase (EC 1.1.1.47) and PQQ-dependent glucose dehydrogenase (EC 1.1.99.17) have, for example, been used as enzymes for blood glucose sensors. These enzymes are advantageous in that they are not influenced by dissolved oxygen. However, NAD(P)-dependent glucose dehydrogenase has poor stability and requires addition of a coenzyme, making the operation complicated; and PQQ-dependent glucose dehydrogenase has drawbacks in that it has poor substrate specificity and acts on saccharides other than glucose, such as maltose and lactose, impairing the accuracy of the measurements.

Patent Document 1 discloses *Aspergillus-derived* flavinlinked glucose dehydrogenase. This enzyme is advantageous in that it has high substrate specificity and is not influenced by dissolved oxygen. The enzyme is reported to have excellent heat stability (heat stability is hereinafter also referred to as heat resistance) since it has a residual activity of about 89% after treatment at 50°C for 15 minutes. However, such a heat stability is insufficient considering that sensor chip preparation processes sometimes involve heat treatment.

Patent Document 1: WO 2004/058958

Disclosure of the Invention

An object of the present invention is to provide an enzyme for blood glucose sensors, the enzyme being free of problems of known enzymes for blood glucose sensors as mentioned above, and being practically more advantageous.

The present inventors, as a result of extensive research to achieve the above object, obtained a glucose dehydrogenase from filamentous fungus of the *Penicillium* genus and found that the glucose dehydrogenases have properties superior to those of known enzymes for measuring blood glucose.

The present invention provides the following items.

1. A eukaryote-derived glucose dehydrogenase which is capable of retaining, after being heat-treated in a liquid state at 55°C for 15 minutes, at least 90% of its activity before the heat treatment.

2. A eukaryote-derived glucose dehydrogenase which is capable of retaining some of its activity after being heat-treated in a liquid state at 60°C for 15 minutes.

3. A eukaryote-derived glucose dehydrogenase which is capable of retaining, after being heat-treated in a liquid state for 60°C for 15 minutes, at least 40% of its activity before the heat treatment.

4. A glucose dehydrogenase according to any one of items 1 to 3, wherein the eukaryote is a filamentous fungus.

5. A glucose dehydrogenase according to item 4, wherein the filamentous fungus is a filamentous fungus of the *Penicillium* genus.

6. A glucose dehydrogenase according to item 5, wherein the filamentous fungus of the *Penicillium* genus is *Penicillium lilacinoechinulatum* or *Penicillium italicum.*

7. A glucose dehydrogenase according to any one of items 1 to 6, whose action on maltose is less than 1% of that on glucose.

8. A glucose dehydrogenase according to item 7, whose action on galactose is less than 2% of that on glucose.

9. A eukaryote-derived glucose dehydrogenase having the following physicochemical properties (a) to (e):

(a) apparent molecular weight determined by gel filtration: about 270 kDa;
(b) optimum reaction temperature: 50°C;

(c) optimum reaction pH: about 6.5;
(d) temperature stability: residual GDH activity of at least 90% after heat treatment at 55°C for 15 minutes, and residual GDH activity of at least 40% after heat treatment at 60°C for 15 minutes; and
(e) pH stability: pH 5.0 to 8.0 (residual activity of at least 90% after treatment at 25°C for 16 hours) .

10. A eukaryote-derived glucose dehydrogenase having the following physicochemical properties (a) to (e):

(a) apparent molecular weight determined by gel filtration: 79 to 93 kDa;
(b) optimum reaction temperature: 60°C;
(c) optimum reaction pH: about 6.5;
(d) temperature stability: residual GDH activity of at least 95% after heat treatment at 55°C for 15 minutes, and residual GDH activity of at least 70% after heat treatment at 60°C for 15 minutes; and
(e) pH stability: pH 5.0 to 8.5 (residual activity of at least 80% after heat treatment at 25°C for 16 hours).

11. A process for producing a glucose dehydrogenase, comprising the steps of culturing a eukaryotic microorganism and extracting and purifying the glucose dehydrogenase according to any one of items 1 to 10.
12. A method for measuring a glucose concentration using a glucose dehydrogenase according to any one of items 1 to 10.
13. A glucose assay kit comprising a glucose dehydrogenase according to any one of items 1 to 10.
14. A glucose sensor comprising a glucose dehydrogenase according to any one of items 1 to 10.

The present invention provides a glucose dehydrogenase that has excellent heat resistance and substrate specificity, and is not influenced by dissolved oxygen.

Brief Description of Drawings

Fig. 1 shows temperature dependences of reaction rates of GDHs of the present invention, expressed as relative activities (%) calculated the highest activity under the temperature conditions employed being defined as 100.
Fig. 2 is pH dependences of reaction rates of GDHs of the present invention, expressed as relative activities (%) calculated with the highest activity under the pH conditions employed being defined as 100.
Fig. 3 is the temperature stability of GDHs of the present invention. The activities after heat treatment at various temperatures are expressed as relative activities (%) calculated with the activity before the heat treatment being defined as 100.
Fig. 4 is the pH stability of a GDH derived from the microorganism deposited under accession no. NBRC6092. The stability at pH 3.3 to 6 using acetate buffer, stability at pH 6 to 7 using PIPES buffer, stability at pH 7 to 8.5 using Tris-hydrochloric acid buffer.
Fig. 5 is the pH stability of a GDH derived from the microorganism deposited under accession no. NBRC32032. The stability at pH 3.3 to 6 using acetate buffer, stability at pH 6 to 7 using PIPES buffer, stability at pH 7 to 8.5 using Tris-hydrochloric acid buffer.
Fig. 6 shows, with respect to a glucose electrode prepared using a GDH of the present invention, the relation between the glucose concentration and response voltage. A response voltage of 1 V output from the detector corresponds to a current of 10 $\mu$A in the glucose electrode.

Best Mode of Carrying Out the Invention

The present invention provides a eukaryote-derived glucose dehydrogenase, which catalyzes the following reaction. D-glucose + electron transfer substance (oxidized form) $\rightarrow$ D-glucono-$\delta$-lactone + electron transfer substance (reduced form)
The glucose dehydrogenase of the present invention has high heat resistance, which distinguishes it from known eukaryote-derived GDHs. Among known eukaryote-derived glucose dehydrogenases, *Aspergillus* terreus-derived GDH is said to have excellent stability. This GDH has, after heat treatment at 55°C for 15 minutes, a residual activity (relative activity calculated with the activity before the heat treatment being defined as 100) of not more than 60%, whereas the GDH of the present invention has a residual activity of not less than 90% after heat treatment at 55°C for 15 minutes. A preferable embodiment of the GDH of the present invention retains some GDH activity after treatment at 60°C for 15 minutes, and a more preferable embodiment has a residual activity of not less than 40% after treatment at 60°C for 15 minutes.

According to the present invention, to test whether the GDH has such heat resistance as described above, heat treatment is performed by heating for 15 minutes the GDH as dissolved in 20 mM potassium phosphate buffer (pH 6.5) so as to exhibit an activity of 1 U/ml. The activity is measured by the method described in the Test Example given hereinafter.

The GDH of the present invention may be derived from any eukaryote. In the present invention, the eukaryote encompasses all of the organisms composed of one or more cells having a nucleus enclosed in a nuclear membrane, and is preferably a filamentous fungus. Preferable examples of filamentous fungi include those of the *Penicillium* genus and *Aspergillus* genus, among which those of the *Penicillium* genus are especially preferable. Particularly preferable examples of filamentous fungi of the *Penicillium* genus include *Penicillium lilacinoechinulatum* and *Penicillium italicum.* Strains of such microorganisms can be easily obtained upon request from organizations storing such strains. Even more preferably, the strains of *Penicillium italicum* and *Penicillium lilacinoechinulatum* are those deposited in the National Institute of Technology and Evaluation, Biological Resource Center, under accession nos. NBRC32032 and NBRC6231, respectively.

The GDH of the present invention also has high substrate specificity, and its action on maltose is less than 1% of that on glucose, and its action on galactose is less than 2% of that on glucose. Action as used herein means the GDH activity at a substrate concentration of 4 mM, and measured by the method described in the Test Example given hereinafter except that the glucose concentration in the reaction mixture used is adjusted to 4 mM.

In another aspect, the present invention provides a eukaryote-derived glucose dehydrogenase having the following properties.

(a) Apparent molecular weight determined by gel filtration: about 270 kDa
(b) Optimum reaction temperature: 50°C
(c) Optimum reaction pH: about 6.5
(d) Temperature stability: residual GDH activity of at least 90% after heat treatment at 55°C for 15 minutes, and residual GDH activity of at least 40% after heat treatment at 60°C for 15 minutes
(e) pH stability: pH 5.0 to 8.0 (residual activity of at least 90% after treatment at 25°C for 16 hours)

The eukaryote from which the GDH of the present invention is derived is not limited as long as it can produce a GDH with the above properties, and is preferably a filamentous fungus, more preferably a filamentous fungus of the *Penicillium* genus, even more preferably *Penicillium lilacinoechinulatum,* and still more preferably the strain deposited in the National Institute of Technology and Evaluation, Biological Resource Center, under accession no. NBRC6231.

The present invention further provides a eukaryote-derived glucose dehydrogenase with the following properties.

(a) Apparent molecular weight determined by gel filtration: 79 to 93 kDa
(b) Optimum reaction temperature: 60°C
(c) Optimum reaction pH: about 6.5
(d) Temperature stability: residual GDH activity of at least 95% after heat treatment at 55°C for 15 minutes, and residual GDH activity of at least 70% after heat treatment at 60°C for 15 minutes
(e) pH stability: pH 5.0 to 8.5 (residual activity of at least 80% after treatment at 25°C for 16 hours)

The eukaryote from which the GDH of the present invention is derived is not limited as long as it can produce a GDH with the above properties, and is preferably a filamentous fungus, more preferably a filamentous fungus of the *Penicillium* genus, even more preferably *Penicillium italicum,* and still more preferably the strain deposited in the National Institute of Technology and Evaluation, Biological Resource Center, under accession no. NBRC32032.

In the present invention, to test whether the GDH has such pH stability as shown above, heat treatment is carried out by heating for 15 minutes the GDH as dissolved in each of 20 mM acetate buffer (pH 4.5 to 6.0), 20 mM PIPES buffer (pH 6.0 to 7.5) and 20 mM Tris hydrochloric acid buffer (pH 7.0 to 8.5) so as to exhibit an activity of 1 U/ml. The activity is measured by the method described in the Test Example given hereinafter.

The gel filtration is carried out under the following conditions. Using a TSK-GEL G3000SW column (7.5 mm x 300 mm, product of Tosoh Corporation) and 50 mM Tris-HCl buffer and 150 mM NaCl buffer (pH 7.5), 25 μl of sample is fractionated at a flow rate of 0.5 ml/min. Based on a calibration curve previously prepared using a standard protein solution, the molecular weight is calculated from the position of peak in the data. The peak position can be determined by monitoring ultraviolet absorbance, or by collecting fractions of the liquid passing through the column and determining the GDH activity peak fraction.

The present invention also provide a process for producing a glucose dehydrogenase, comprising the steps of culturing a eukaryotic microorganism and extracting and purifying a GDH with the above properties. Eukaryotic microorganisms include filamentous fungi, yeasts and eukaryotic algae. The eukaryotic microorganism for use in the present invention is not limited as long as it can produce a GDH with the above properties, and is preferably a filamentous fungus, more preferably a filamentous fungus of the *Penicillium* genus, and even more preferably *Penicillium lilacinoechinulatum* or *Penicillium italicum.* Strains belonging to these genus or species can be used in the present invention. Particularly preferable examples are *Penicillium lilacinoechinulatum* NBRC6231 and *Penicillium italicum* NBRC32032.

The medium for culturing the microorganism is not limited as long as it allows the microorganism to grow and produce the GDH according to the present invention. The medium preferably contains a carbon source, inorganic nitrogen source and/or organic nitrogen source, as required for the growth of the microorganism, and more preferably is a liquid medium that can be suitably aerated and agitated. When using a liquid medium, examples of usable carbon sources include glucose, dextran, soluble starch, sucrose, etc., and examples of usable nitrogen sources include ammonium salts, nitrates, amino acids, corn steep liquor, peptones, casein, meat extracts, defatted soybeans, potato extracts, etc. The medium may contain, as required, other nutrients (e.g., calcium chloride, sodium dihydrogenphosphate, magnesium chloride and other mineral salts; vitamins; etc.).

The culturing may be carried out by methods known in the art. For example, a liquid medium containing such nutrients as mentioned above is inoculated with spores or growing cells of the microorganism, and allowed to stand, or aerated and agitated, to proliferate the cells. Preferably, the microorganism is cultured with aeration and agitation. The liquid medium preferably has a pH of 5 to 9, and more preferably a pH of 6 to 8. The culturing temperature is usually 14 to 42°C, and more preferably 20°C to 40°C. The culturing is usually continued for 14 to 144 hours. Preferably, the culturing is terminated when the GDH expression amount reaches a maximum under the culture conditions employed. Such a point of time can be determined by sampling the liquid medium to measure the GDH activity in the medium and thereby monitor the change in the activity. When the increase of GDH activity with time has stopped, that point of time is taken as the peak and the culturing is terminated.

The GDH can be extracted from the culture medium as follows. For collecting GDH accumulated in cells, only the cells are recovered by centrifugation, filtration or another method, and re-suspended in a solvent, which is preferably water or a buffer. The re-suspended cells are disrupted by a known method to thereby extract the GDH from the cells into the solvent. Usable cell disruption methods include use of lytic enzymes and physical disruption methods. Usable lytic enzymes are not limited as long as they are capable of digesting the cell walls of fungi, and include, for example, "Lyticase" (product of Sigma). Examples of physical disruption methods include ultrasonic disruption, glass bead disruption, French-press disruption, etc. The solution after the disruption treatment is subjected to centrifugation or filtration to remove the residue, to thereby obtain a crude GDH extract.

Further, in the present invention, the culturing may be carried out using a solid medium. Preferably, after suitably controlling the temperature, humidity and other factors, an eukaryotic microorganism that can produce the GDH of the present invention is grown on bran of wheat or the like. For growing the microorganism, the culture medium may be allowed to stand, or mixed with the microorganism by, for example, agitation. The GDH is extracted by adding a solvent, which is preferably water or a buffer, to the culture to thereby dissolve the GDH, followed by centrifugation or filtration to remove solids such as the cells and bran.

The GDH can be purified by a suitable combination of conventional separation techniques selected according to the fraction having GDH activity. That is, the GDH extract can be subjected to known separation technique(s) selected from, for example, salting-out, solvent precipitation, dialysis, ultrafiltration, gel filtration, undenatured PAGE, SDS-PAGE, ion exchange chromatography, hydroxyapatite chromatography, affinity chromatography, reverse phase high performance liquid chromatography, isoelectric focusing, etc. Stabilizers or other substances can be added to the extracted or purified GDH solution. Examples of substances that can be added include mannitol, trehalose, sucrose, sorbitol, erythritol, glycerol and other sugars and sugar alcohols; glutamic acid, arginine and other amino acids; bovine serum albumin, ovalbumin, chaperons and other proteins and peptides; etc.

The GDH of the present invention can be provided in a liquid form, and may be lyophilized, vacuum-dried or spray-dried to form a powder. For forming a powder, the GDH can be used as a solution in a buffer or the like, and preferably, a sugar, sugar alcohol, amino acid, protein, peptide and/or the like is added as an excipient and/or stabilizer. The powder formed may be granulated.

The composition of the buffer used for extraction, purification and powder formation of the GDH is not limited. Buffers with a buffering capacity at pH 5 to 8 are preferable. Examples of such buffers include boric acid, Tris-hydrochloric acid, potassium phosphate and the like; and Good's buffers such as BES, Bicine, Bis-Tris, CHES, EPPS, HEPES, HEPPSO, MES, MOPS, MOPSO, PIPES, POPSO, TAPS, TAPSO, TES, Tricine, etc.

According to the present invention, glucose can be measured by various methods as described below.

Glucose assay kit

The present invention also provides a glucose assay kit comprising the GDH of the present invention. The glucose assay kit of the present invention comprises the GDH of the present invention in an amount sufficient to perform the assay at least once. Typically, the kit comprises, in addition to the GDH of the present invention, a buffer, mediator, glucose standard solution for calibration curve preparation, and an instruction manual, as required for the assay. The GDH of the present invention can be provided in various forms: for example, as a lyophilized reagent or a solution in a suitable preservation solution.

Glucose sensor

The present invention also provides a glucose sensor using the GDH of the present invention. The electrode is a carbon electrode, gold electrode, platinum electrode or the like, on which the enzyme of the present invention is immobilized. The enzyme can be immobilized by use of a crosslinking reagent, encapsulation in a polymeric matrix, covering with a dialysis membrane, use of a photocrosslinkable polymer, electrically conductive polymer or oxidation-reduction polymer, or another method. Alternatively, the enzyme, together with a mediator, may be immobilized in a polymer, or adsorbed and immobilized on an electrode. These methods may be used in combination. Typically, the GDH of the present invention is immobilized on a carbon electrode using glutaraldehyde, and thereafter the glutaraldehyde is blocked by treatment with an amine-containing reagent.

The glucose concentration can be measured as follows. A buffer is placed in a constant-temperature cell, a mediator is added, and the temperature of the mixture in the cell is kept constant. An electrode having the GDH of the present invention immobilized thereon is used as a working electrode, and a counter electrode (e.g., platinum electrode) and a reference electrode (e.g., Ag/AgCl electrode) are used. A predetermined voltage is applied to the carbon electrode, and after the current has steadied, a sample containing glucose is added and the increase of current is measured. Based on a calibration curve prepared using glucose solutions with standard concentrations, the glucose concentration in the sample can be calculated.

The mediator for use in the glucose measuring composition, glucose assay kit, glucose sensor and glucose measuring method according to the present invention is not limited. Examples of preferable mediators include 2,6-dichlorophenol-indophenol (abbreviated as DCPIP), ferrocene and derivatives thereof (e.g., potassium ferricyanide, phenazine methosulfate, etc.) and the like. Such mediators are commercially available.

Test Example

In the present invention, glucose dehydrogenase activity is measured under the following conditions.

<Reagent>

50 mM PIPES buffer at pH 6.5 (containing 0.1% Triton X-100) 14 mM 2,6-dichlorophenol-indophenol (DCPIP) solution 1 M D-glucose solution

The PIPES buffer (15.8 ml), DCPIP solution (0.2 ml) and D-glucose solution (4 ml) are mixed and used as a reaction reagent.

<Measurement conditions>

2.9 ml of the reaction reagent is preheated at 37°C for 5 minutes. 0.1 ml of a GDH solution is added, and after gentle stirring, the change in absorbance at 600 nm is recorded for 5 minutes with a spectrophotometer maintained at 37°C, using water as a control. The absorbance change per minute ($\Delta OD_{TEST}$) is determined from the linear portion. As a blank test, the absorbance change per minute ($\Delta OD_{BLANK}$) is determined in the same manner as above except that a solvent of the GDH solution is added to the reagent in place of the GDH solution. From the values thus obtained, the GDH activity is calculated by the following equation 1. One unit (U) of the GDH activity is defined as the amount of enzyme that reduces 1 $\mu$mol of DCPIP per minute in the presence of 200 mM D-glucose.

[Equation 1]

$$\text{Activity (U/ml)} = \frac{-(\Delta OD_{TEST} - \Delta OD_{BLANK}) \times 3.0 \times \text{Dilution Factor}}{16.3 \times 0.1 \times 1.0}$$

In the equation, 3.0 is the combined amount (ml) of the reaction reagent and enzyme solution, 16.3 is the millimolar extinction coefficient ($cm^2/\mu mol$) under the activity measurement conditions, 0.1 is the amount (ml) of the enzyme solution, and 1.0 is the optical path length (cm) of the cell.

Example

The following examples illustrate the present invention in more detail, without limiting the invention thereto.

<Example 1>

Preparation of *Penicillium* fungi-derived GDH

*Penicillium lilacinoechinulatum* NBRC6231 and *Penicillium italicum* NBRC32032 (purchased from the National Institute of Technology and Evaluation) were used as GDH-producing fungi. Specimens of these fungi dried from the liquid state were inoculated on potato dextrose agar medium (product of Difco) and incubated at 25°C to restore the fungi. The restored mycelia on the plates were collected together with the agar and suspended in filter-sterilized water. Six liters of a production culture medium (1% malt extract, 1.5% soybean peptide, 0.1% $MgSO_4 \cdot 7H_2O$, 2% glucose, pH 6.5) was prepared in two 10-liter capacity jar fermenters. After autoclaving at 120°C for 15 minutes, the mycelia suspensions were each placed in one of the fermenters, and cultured at a temperature of 30°C, an air flow rate of 2 liters/minute, and a stirring rate of 380 rpm. Sixty-four hours after the start of culturing, the culturing was stopped and the cells of each strain were recovered on filter paper by suction filtration using a Nutsche filter. The cells were re-suspended in 3 liters of a 20mM potassium phosphate buffer (pH 6.5), and disrupted by French-press at a pressure of 130 MPa. Each cell lysate was placed in a 500 ml-capacity centrifugation tube and centrifuged at 8000 rpm for 15 minutes using a Hitachi high-speed refrigerated centrifuge to precipitate a residue. The supernatant was concentrated to 1/10 volume by using an ultrafiltration hollow fiber module with a molecular weight cut-off of 10,000. Ammonium sulfate was added to each concentrate and dissolved to achieve a final concentration of 60% saturation (456 g/L). Subsequently, the resulting solution was centrifuged at 8000 rpm for 15 minutes using a Hitachi high-speed refrigerated centrifuge to precipitate a residue. The supernatant was then adsorbed on an Octyl-Sepharose column and subjected to gradient elution with ammonium sulfate at concentrations of 0.6 to 0.0 saturation to recover fractions with GDH activity. Each obtained GDH solution was desalted by gel filtration using a G-25 Sepharose column to collect a protein fraction, and ammonium sulfate at a concentration corresponding to 0.6 saturation was added and dissolved in the desalted solution. The resulting solution was adsorbed on a Phenyl-Sepharose column and subjected to gradient elution with ammonium sulfate at concentrations of 0.6 to 0.0 saturation to recover fractions with GDH activity. This was heated at 50°C for 45 minutes and centrifuged to obtain a supernatant. The solution thus obtained was used as a purified GDH sample.

<Example 2>

Prediction of molecular weight

25 $\mu$L of each of the purified NBRC6231- and NBRC32032-derived GDH solutions obtained in Example 1 were charged on TSK-GEL G3000SW (7.5mm x 300mm) (product of Tosoh Corporation) buffered with 50mM Tris-HCl (pH 7.5) and fractionated at a flow rate of 0.5 ml/mins. During elution of NBRC6231, absorbance at 280nm was monitored to determine the elution time from the position of the peak. In the case of NBRC32032, the eluate was collected by a fraction collector, and GDH activity of each fraction was determined according to Test Example 1 to determine the peak fraction, and the elution time was calculated therefrom. Based on the determined elution times, the molecular weight of GDH proteins was calculated from a calibration curve pre-prepared using a reference protein solution (MW-Marker (MW 12,400 to 290,000), product of Oriental Yeast Co., Ltd.). From the results, the predicted molecular weight of NBRC6241-derived GDH was about 270 kDa, and that of NBRC32032-derived GDH was in the range of 79 to 93 kDa.

<Example 3>

Optimal reaction temperature

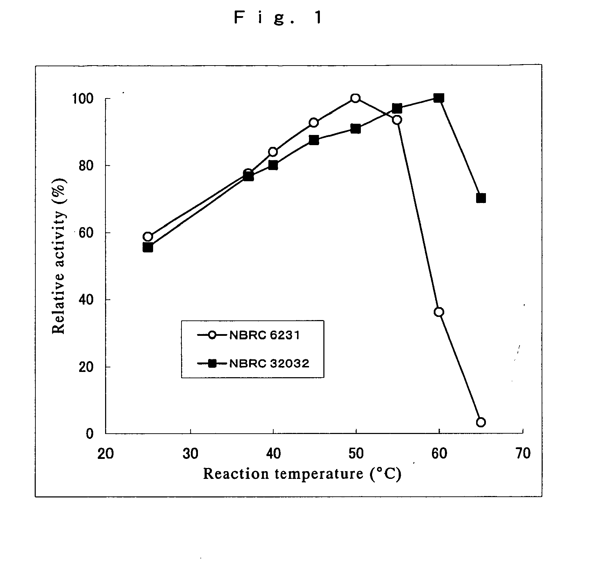

To find the optimal reaction temperatures for the purified NBRC6231 and NBRC32032 solutions obtained in Example 1, measurements were performed according to the same procedures as in the Test Example above except using the temperatures of 25, 37, 40, 45, 50, 55, 60 and 65°C as preliminary heating temperature and temperature of the reaction reagent during the reaction, and the activities under these conditions were calculated. Fig. 1 is a graph showing relative activities calculated with the highest activity under such conditions being defined as 100. The results show that under the above conditions, the optimal reaction temperature for NBRC6231-derived GDH is in the range of higher than 45°C and lower than 55°C, i.e., about 50°C, and the optimal reaction temperature for NBRC32032-derived GDH is in the range of higher than 55°C and lower than 60°C, i.e., about 60°C.

<Example 4>

Optimal reaction pH

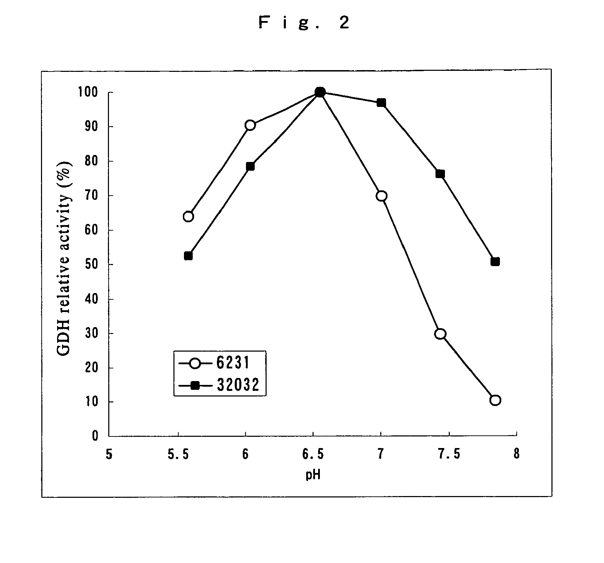

To find the optimal pHs for the purified NBRC6231 and NBRC32032 solutions obtained in Example 1, reaction reagents were prepared using the same components as in the Test Example above except for using 50 mM potassium phosphate buffer solutions at pHs in the range of 5.5 to 8.0 in place of the PIPES buffer solution, and activity measurement was performed according to the same procedures as in the Test Example. Fig. 2 is a graph showing relative activities under the respective pH conditions calculated with the highest activity under such pH conditions being defined as 100. Both NBRC 6231-derived GDH and NBRC 32032-derived GDH exhibited the highest activity at a pH of around 6.5. These results show that under the above conditions, the optimal reaction pH for both NBRC6231-derived GDH and NBRC32032-derived GDH is in the range of higher than 6.0 and lower than 7.0, i.e., about 6.5.

<Example 5>

Temperature stability

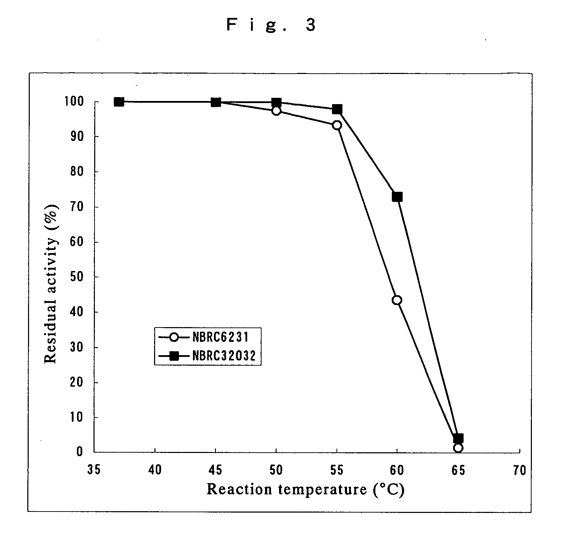

To check the temperature stabilities of the purified NBRC6231 and NBRC32032 solutions obtained in Example 1, each GDH solution was diluted with a 20 mM potassium phosphate buffer solution (pH 6.5) to an activity of 1 U/ml. Each dilute solution was subjected to heat treatment at temperatures in the range of 37 to 65°C for 15 minutes using a heating bath. The activities before and after the treatment were compared. Fig. 3 is a graph showing the residual activity after heat treatment compared to the activity before heat treatment. The residual activity % of NBRC 6231-derived GDH was 93% after treatment at 55 °C, and 44% after treatment at 60°C. The residual activity % of NBRC 32032-derived GDH was 98% after treatment at 55 °C, and 73% after treatment at 60°C.

<Example 6>

pH stability

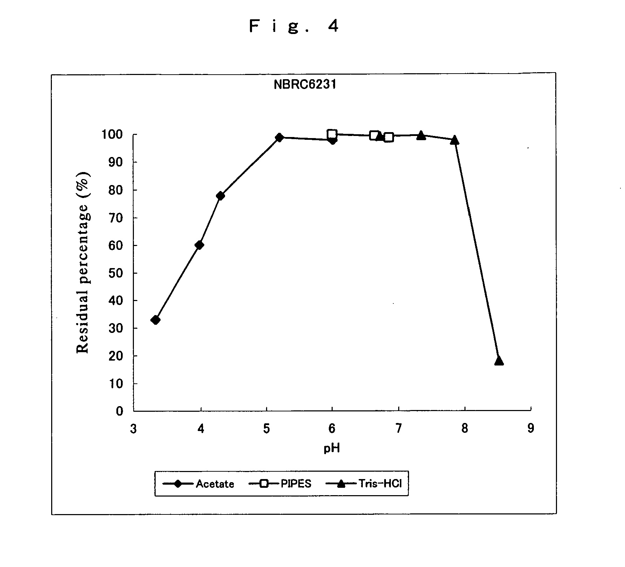

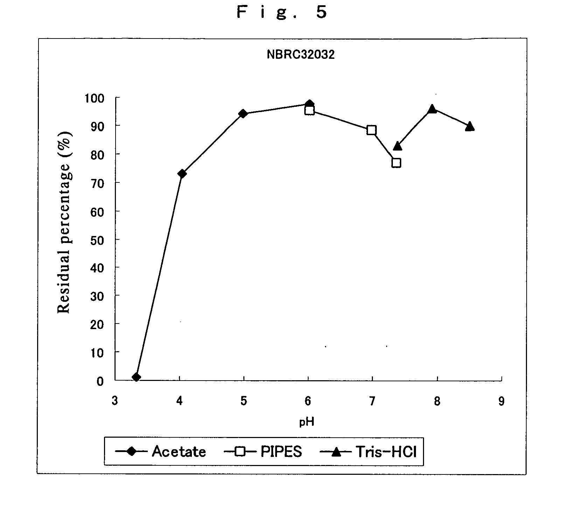

To check the pH stabilities of the purified NBRC6231 and NBRC32032 solutions obtained in Example 1, buffer solutions at pHs in the range of 3.3 to 8.5 (acetic acid buffers at pH 3 to 6, PIPES buffers at pH 6 to 7, and Tris hydrochloric acid buffers at pH 7 to 8.5) were prepared. Each GDH was diluted with these buffer solutions to an enzyme concentration of 1U/ml. Each diluted solution was incubated at 25°C for 16 hours and the activities before and after incubation were compared. Fig. 4 is a graph showing the residual activity % of NBRC6231-derived GDH after incubation compared to the activity before incubation. Fig. 5 is a graph showing the residual activity % of NBRC32032-derived GDH after incubation compared to the activity before incubation. NBRC6231-derived GDH had a residual activity of at least 80% at pH 5 to 8.5 and exhibited excellent stability. NBRC32032-derived GDH exhibited a residual activity of at least 80% at pH 5 to 8.5, except for one case: when the buffer was PIPES, it exhibited a residual activity of 77% at pH 7.4.

<Example 7>

Km value for D-glucose

Among the components of the reaction reagent described in the Test Example, the concentration of D-glucose was varied within a range not higher than 200 mM. Using these reaction agents, NBRC6231- and NBRC32032-derived GDH activities were determined. The Km values were calculated according to the Lineweaver-Burk plot. The calculation results show that the Km value of NBRC6231-derived GDH was 13 mM, and that of NBRC32032-derived GDH was 1.8 mM.

<Example 8>

Application to glucose electrode

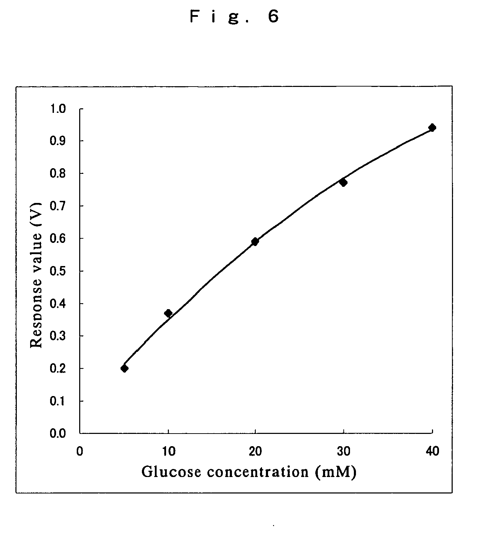

0.5 g of carbon graphite was placed in a mortar. After addition of 0.3 mL of liquid paraffin, the mixture was ground with a pestle to a carbon paste. The carbon paste thus obtained was applied onto a platinum electrode. Further, 10 μL of a purified GDH solution (Deposit No. NBRC6231-derived, 500 U/mL) prepared according to Example 1 was added, followed by air drying at room temperature for 30 minutes. A cellulose semipermeable membrane with a molecular weight cut-off of 8,000 was placed on the air-dried enzymatic electrode and fixed with a plastic 0-ring. The glucose electrode thus obtained was used after immersion in an ice-cooled 50mM potassium phosphate buffer (pH 7.0) for 30 minutes. Twenty ml of reaction mixture was placed in a cuvette maintained at 25°C, and a glucose electrode (working electrode), a platinum electrode as the counter electrode, and an Ag/AgCl electrode as a reference electrode were immersed therein and a voltage of +0.35V was applied. After the current became constant, glucose was added. The current value in response thereto was monitored. The detector was used to output by converting a current value of 1 $\mu$A to a voltage value of O.1V, and graph time-dependent output voltage value changes. The degree of voltage increase above the background value to the constant value achieved after addition of glucose was defined as the response value (V). The concentration of glucose added was varied to final concentrations in the range of 5 to 40 mM. Fig. 6 is a graph made by plotting the response values obtained with respective glucose concentrations. A glucose concentration-dependent response value increase was observed, thus confirming that the GDH of the invention can be used for quantitative determination of glucose and can be used as a glucose sensor.

Industrial Applicability

The present invention provides compositions and methods for measuring glucose. These compositions and methods can be used for glucose assay kits and glucose assay sensors.

**Claims**

1. A eukaryote-derived glucose dehydrogenase which is capable of retaining, after being heat-treated in a liquid state at 55°C for 15 minutes, at least 90% of its activity before the heat treatment.

2. A eukaryote-derived glucose dehydrogenase which is capable of retaining some of its activity after being heat-treated in a liquid state at 60°C for 15 minutes.

3. A eukaryote-derived glucose dehydrogenase which is capable of retaining, after being heat-treated in a liquid state for 60°C for 15 minutes, at least 40% of its activity before the heat treatment.

4. A glucose dehydrogenase according to any one of claims 1 to 3, wherein the eukaryote is a filamentous fungus.

5. A glucose dehydrogenase according to claim 4, wherein the filamentous fungus is a filamentous fungus of the *Penicillium* genus.

6. A glucose dehydrogenase according to claim 5, wherein the filamentous fungus of the *Penicillium* genus is *Penicillium lilacinoechinulatum* or *Penicillium italicum.*

7. A glucose dehydrogenase according to any one of claims 1 to 6, whose action on maltose is less than 1% of that on glucose.

8. A glucose dehydrogenase according to claim 7, whose action on galactose is less than 2% of that on glucose.

9. A eukaryote-derived glucose dehydrogenase having the following physicochemical properties (b) to (e):

   (b) optimum reaction temperature: 50°C;
   (c) optimum reaction pH: about 6.5;
   (d) temperature stability: residual GDH activity of at least 90% after heat treatment at 55°C for 15 minutes, and residual GDH activity of at least 40% after heat treatment at 60°C for 15 minutes; and
   (e) pH stability: pH 5.0 to 8.0 (residual activity of at least 90% after treatment at 25°C for 16 hours).

10. A eukaryote-derived glucose dehydrogenase having the following physicochemical properties (b) to (e):

   (b) optimum reaction temperature: 60°C;
   (c) optimum reaction pH: about 6.5;
   (d) temperature stability: residual GDH activity of at least 95% after heat treatment at 55°C for 15 minutes, and residual GDH activity of at least 70% after heat treatment at 60°C for 15 minutes; and
   (e) pH stability: pH 5.0 to 8.5 (residual activity of at least 80% after heat treatment at 25°C for 16 hours).

**11.** A process for producing a glucose dehydrogenase, comprising the steps of culturing a eukaryotic microorganism and extracting and purifying a glucose dehydrogenase according to any one of claims 1 to 10.

**12.** A method for measuring a glucose concentration using a glucose dehydrogenase according to any one of claims 1 to 10.

**13.** A glucose assay kit comprising a glucose dehydrogenase according to any one of claims 1 to 10.

**14.** A glucose sensor comprising a glucose dehydrogenase according to any one of claims 1 to 10.

**Amended claims in accordance with Rule 86(2) EPC.**

**1.** A glucose dehydrogenase derived from *Penicillium lilacinoechinulatum* having the following physicochemical properties (a) to (e):

(a) apparent molecular weight determined by gel filtration: about 270 kDa;
(b) optimum reaction temperature: 50°C;
(c) optimum reaction pH: about 6.5;
(d) temperature stability: residual GDH activity of at least 90% after heat treatment at 55°C for 15 minutes, and residual GDH activity of at least 40% after heat treatment at 60°C for 15 minutes; and
(e) pH stability: pH 5.0 to 8.0 (residual activity of at least 90% after treatment at 25°C for 16 hours).

**2.** A glucose dehydrogenase derived from *Penicillium italicum* having the following physicochemical properties (a) to (e):

(a) apparent molecular weight determined by gel filtration: about 79 to 93 kDa;
(b) optimum reaction temperature: 60°C;
(c) optimum reaction pH: about 6.5;
(d) temperature stability: residual GDH activity of at least 95% after heat treatment at 55°C for 15 minutes, and residual GDH activity of at least 70% after heat treatment at 60°C for 15 minutes; and
(e) pH stability: pH 5.0 to 8.5 (residual activity of at least 80% after heat treatment at 25°C for 16 hours).

**3.** The glucose dehydrogenase of claim 1 or 2, wherein action on maltose is less than 1% of that on glucose.

**4.** The glucose dehydrogenase of claim 3, wherein action on galactose is less than 2% of that on glucose.

**5.** A process for producing a glucose dehydrogenase, comprising the steps of culturing a eukaryotic microorganism and extracting and purifying a glucose dehydrogenase according to any one of claims 1 to 4.

**6.** A method for measuring a glucose concentration using a glucose dehydrogenase according to any one of claims 1 to 4.

**7.** A glucose assay kit comprising a glucose dehydrogenase according to any one of claims 1 to 4.

**8.** A glucose sensor comprising a glucose dehydrogenase according to any one of claims 1 to 4.

F i g . 1

F i g .  2

F i g . 3

F i g . 4

F i g .  5

F i g . 6

**European Patent Office**

**EUROPEAN SEARCH REPORT**

Application Number

EP 06 00 6698

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ELZAINY T A ET AL: "Nonphosphorylated glucose catabolism by Penicillium citrinum" ANNALI DI MICROBIOLOGIA ED ENZIMOLOGIA, vol. 43, no. 2, 1993, pages 169-179, XP001246849 ISSN: 0003-4649 * page 171 - page 173; figure 1 * | 1-5,7,9, 10 | INV. C12N9/04 C07K14/385 C12Q1/00 |
| Y | | 11-14 | |
| Y | EP 1 584 675 A (IKEDA FOOD RESEARCH CO. LTD) 12 October 2005 (2005-10-12) * claims 1-15; examples 4-6 * | 11-14 | |
| A | SCOGNAMIGLIO VIVIANA ET AL: "Protein-based biosensors for diabetic patients." JOURNAL OF FLUORESCENCE. SEP 2004, vol. 14, no. 5, September 2004 (2004-09), pages 491-498, XP002384635 ISSN: 1053-0509 * page 493, column 2 - page 494 * | 11-14 | |
| | | | **TECHNICAL FIELDS SEARCHED (IPC)** |
| A | US 4 877 733 A (TAKAHASHI ET AL) 31 October 1989 (1989-10-31) * figures 3,4; table 2 * | 1-3,7,8 | C12N C07K C12Q |
| A | BELENKII B G ET AL: "[AN ANTIBIOTIC FROM A NEW TYPE OF PENICILLIUM WITH THE GLUCOSE DEHYDROGENASE ACTIVITY.]" ANTIBIOTIKI. JUL 1964, vol. 18, July 1964 (1964-07), pages 602-603, XP009067534 ISSN: 0003-5637 * abstract * | 1-4 | |

The present search report has been drawn up for all claims

| Place of search | Date of completion of the search | Examiner |
|---|---|---|
| Munich | 20 June 2006 | Deleu, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**                    EP 06 00 6698

This annex lists the patent family members relating to the patent documents cited in the above-mentioned European search report.
The members are as contained in the European Patent Office EDP file on
The European Patent Office is in no way liable for these particulars which are merely given for the purpose of information.

20-06-2006

| Patent document cited in search report | | Publication date | Patent family member(s) | | Publication date |
|---|---|---|---|---|---|
| EP 1584675 | A | 12-10-2005 | AU | 2003296186 A1 | 22-07-2004 |
| | | | CA | 2511656 A1 | 15-07-2004 |
| | | | CN | 1726277 A | 25-01-2006 |
| | | | WO | 2004058958 A1 | 15-07-2004 |
| | | | US | 2006063217 A1 | 23-03-2006 |
| US 4877733 | A | 31-10-1989 | DE | 3714544 A1 | 12-11-1987 |
| | | | FR | 2598156 A1 | 06-11-1987 |
| | | | IT | 1204549 B | 03-03-1989 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004058958 A